# EUROPÄISCHE PATENTSCHRIFT

(11) **EP 1 417 173 B1**
(45) Veröffentlichungstag und Bekanntmachung des Hinweises auf die Patenterteilung: **26.09.2007**
(21) Anmeldenummer: 02787109.4
(22) Anmeldetag: 03.07.2002
(51) Int. Cl.: C07D 213/85, C07D 417/12, A61K 31/4427, A61P 9/00, A61P 15/00, A61P 3/00, A61P 29/00

(54) **SUBSTITUIERTE 2-THIO-3,5-DICYANO-4-PHENYL-6-AMINOPYRIDINE UND IHRE VERWENDUNG ALS ADENOSINREZEPTOR-SELEKTIVE LIGANDEN**
SUBSTITUTED 2-THIO-3, 5-DICYANO-4-PHENYL-6-AMINOPYRIDINES AND THEIR USE AS ADENOSINE RECEPTOR-SELECTIVE LIGANDS
2-THIO-3,5-DICYANO-4-PHENYL-6-AMINOPYRIDINES SUBSTITUEES ET LEUR UTILISATION EN TANT QUE LIGANDS SELECTIFS DE RECEPTEUR D'ADENOSINE

(30) Priorität: 16.07.2001 DE 10134481
(43) Veröffentlichungstag der Anmeldung: 12.05.2004
(73) Patentinhaber: Bayer HealthCare AG, 51368 Leverkusen (DE)
(72) Erfinder: ROSENTRETER, Ulrich, 42349 Wuppertal (DE); KRÄMER, Thomas, 42111 Wuppertal (DE); SHIMADA, Mitsuyuki, Nara, 630-8323 (JP); HÜBSCH, Walter, 42113 Wuppertal (DE); DIEDRICHS, Nicole, 42103 Wuppertal (DE); KRAHN, Thomas, 58135 Hagen (DE); HENNINGER, Kerstin, 42115 Wuppertal (DE); STASCH, Johannes-Peter, 42651 Solingen (DE)
(86) Internationale Anmeldenummer: PCT/EP2002/007324
(87) Internationale Veröffentlichungsnummer: WO 2003/008384

(56) Entgegenhaltungen:
- WO-A-01/25210
- POULSEN S-A ET AL: "ADENOSINE RECEPTORS: NEW OPPORTUNITIES FOR FUTURE DRUGS" BIOORGANIC & MEDICINAL CHEMISTRY, ELSEVIER SCIENCE LTD, GB, Bd. 6, 1998, Seiten 619-641, XP000985735 ISSN: 0968-0896 in der Anmeldung erwähnt

## Beschreibung

Die vorliegende Erfindung betrifft substituierte 2-Thio-3,5-dicyano-4-phenyl-6-aminopyridine, ein Verfahren zu ihrer Herstellung und ihre Verwendung als Arzneimittel.

Adenosin, ein Nucleosid aus Adenin und D-Ribose, ist ein endogener Faktor mit zellprotektiver Wirksamkeit, insbesondere unter zellschädigenden Bedingungen mit begrenzter Sauerstoff- und Substratversorgung, wie z.B. bei Ischämie in verschiedensten Organen (z.B. Herz und Gehim).

Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf die Herzfrequenz, auf die Ausschüttung von Neurotransmittern und auf die Lymphozyten-Differenzierung.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Als "Adenosinrezeptor-selektive Liganden" werden erfindungsgemäß solche Substanzen bezeichnet, die selektiv an einen oder mehrere Subtypen der Adenosinrezeptoren binden und dabei entweder die Wirkung des Adenosin nachahmen (Adenosin-Agonisten) oder dessen Wirkung blockieren (Adenosin-Antagonisten) können.

Adenosinrezeptor-selektive Liganden lassen sich nach ihrer Rezeptorselektivität in verschiedene Klassen einteilen, so z.B. in Liganden, die selektiv an die A1- oder die A2-Rezeptoren des Adenosin binden, bei letzteren auch beispielsweise solche, die selektiv an die A2a- oder die A2b-Rezeptoren des Adenosin binden. Auch sind Adenosinrezeptor-Liganden möglich, die selektiv an mehrere Subtypen der Adenosinrezeptoren binden, so z.B. Liganden, die selektiv an die A1- und an die A2-, jedoch nicht an die A3-Rezeptoren des Adenosin binden.

Die zuvor genannte Rezeptor-Selektivität lässt sich beispielsweise bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis." in J. Biol. Chem. 267 (1992) Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells" in Naunyn Schmiedebergs Arch. Pharmacol. 357 (1998) Seiten 1-9, deren Offenbarung hiermit im vollen Umfang durch Bezugnahme eingeschlossen ist).

Bei den aus dem Stand der Technik bekannten Adenosinrezeptor-Liganden handelt es sich überwiegend um Derivate auf Basis des natürlichen Adenosins (S.-A. Poulsen und R. J. Quinn, "Adenosine receptors: new opportunities for future drugs" in Bioorganic and Medicinal Chemistry 6 (1998) Seiten 619-641). Diese aus dem Stand der Technik bekannten Adenosin-Liganden haben jedoch meistens den Nachteil, dass sie schwächer wirksam sind als das natürliche Adenosin oder nach oraler Applikation nur sehr schwach oder gar nicht wirksam sind. Deshalb werden sie überwiegend nur für experimentelle Zwecke verwendet.

Darüber hinaus sind aus WO 01/25210 2-Thio-3,5-dicyano-4-aryl-6-aminopyridine bekannt, die den erfindungsgemäßen Verbindungen strukturell ähnlich sind. Die dort beschriebenen Verbindungen haben allerdings unvorteilhafte pharmakokinetische Eigenschaften, insbesondere besitzen Sie nur eine geringe Bioverfügbarkeit nach oraler Gabe.

Aufgabe der vorliegenden Erfindung ist nunmehr die Auffindung bzw. Bereitstellung von Verbindungen, die die Nachteile des Standes der Technik vermeiden, d.h. insbesondere eine verbesserte Bioverfügbarkeit besitzen.

Die vorliegende Erfindung betrifft Verbindungen der Formel (I) 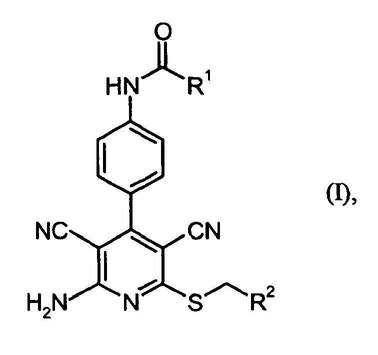 worin
- R¹: (C₁-C₄)-Alkyl, (C₁-C₄)-Alkoxy, Mono- oder Di-(C₁-C₄)-alkylamino bedeutet
und
- R²: Pyridyl der durch Halogen, Amino oder (C₁-C₄)-Alkyl substituiert sein kann, bedeutet
und ihre Salze, Hydrate, Hydrate der Salze und Solvate.

Die Verbindungen der Formel (I) können in Abhängigkeit vom Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen. Gleichermaßen betrifft die vorliegende Erfindung auch die übrigen Tautomeren der Verbindungen der Formel (I) und deren Salze.

Salze der Verbindungen der Formel (I) können physiologisch unbedenkliche Salze der erfindungsgemäßen Stoffe mit Mineralsäuren, Carbonsäuren oder Sulfonsäuren sein.

Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Trifluoressigsäure, Essigsäure, Propionsäure, Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Als Hydrate bzw. Solvate werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Außerdem umfasst die Erfindung auch Prodrugs der erfindungsgemäßen Verbindungen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Im Rahmen der vorliegenden Erfindung haben die Substituenten, soweit nicht anders angegeben, die folgende Bedeutung:
Halogen steht im allgemeinen für Fluor, Chlor, Brom oder Iod. Bevorzugt sind Fluor, Chlor oder Brom. Ganz besonders bevorzugt sind Fluor oder Chlor.
(C₁-C₄)-Alkyl steht im allgemeinen für einen geradkettigen oder verzweigten Alkylrest mit 1 bis 4 Kohlenstoffatomen. Beispielsweise seien genannt: Methyl, Ethyl, n-Propyl, Isopropyl, n-Butyl, sec-Butyl, Isobutyl und tert.-Butyl.
(C₁-C₄)-Alkoxy steht im allgemeinen für einen geradkettigen oder verzweigten Alkoxyrest mit 1 bis 4 Kohlenstoffatomen. Beispielsweise seien genannt: Methoxy, Ethoxy, n-Propoxy, Isopropoxy, n-Butoxy, sec-Butoxy, Isobutoxy, tert.-Butoxy.
Mono- oder Di-(C₁-C₄)-alkylamino steht im allgemeinen für eine Amino-Gruppe mit einem oder mit zwei gleichen oder verschiedenen geradkettigen oder verzweigten Alkylsubstituenten, die jeweils 1 bis 4 Kohlenstoffatome aufweisen. Beispielsweise seien genannt: Methylamino, Ethylamino, n-Propylamino, Isopropylamino, t-Butylamino, *N,N-*Dimethylamino, *N,N*-Diethylamino, *N*-Ethyl-*N*-methylamino, *N*-Methyl-*N-*n-propylamino, *N-*Isopropyl-*N-*n*-*propylamino und *N*-t-Butyl-*N*-methylamino.

Bevorzugt sind Verbindungen der Formel (I),
worin
- R¹: Methyl, Ethyl, n-Propyl, Isopropyl, n-Butyl, sec-Butyl, Isobutyl oder tert.-Butyl bedeutet
und
- R²: 2-Pyridyl, der durch Chlor, Amino oder Methyl substituiert sein kann, bedeutet
und ihre Salze, Hydrate, Hydrate der Salze und Solvate.

Besonders bevorzugt sind Verbindungen der Formel (I), worin R¹ (C₁-C₄)-Alkyl bedeutet, und ihre Salze, Hydrate, Hydrate der Salze und Solvate.

Ebenfalls besonders bevorzugt sind Verbindungen der Formel (I), worin R² unsubstituiertes Pyridyl bedeutet, und ihre Salze, Hydrate, Hydrate der Salze und Solvate.

Ebenfalls besonders bevorzugt ist die Verbindung mit der folgenden Formel 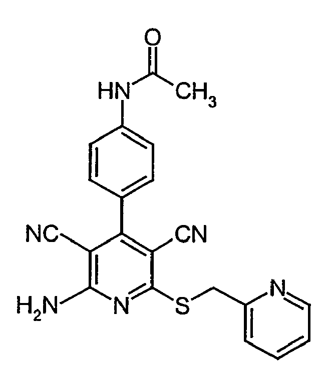 und ihre Salze, Hydrate, Hydrate der Salze und Solvate.

Gegenstand der vorliegenden Erfindung ist auch ein Verfahren zur Herstellung der Verbindungen der Formel (I), dass dadurch gekennzeichnet ist, dass man
Verbindungen der Formel (II) 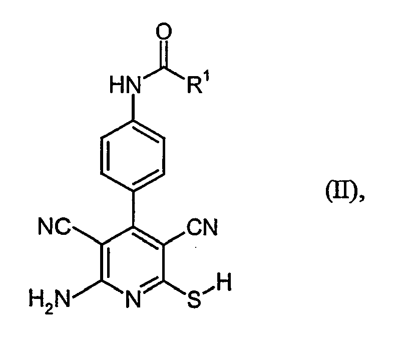 worin
- R¹: die zuvor angegebene Bedeutung hat,
mit Verbindungen der Formel (III)

R²-CH₂-X (III),

worin
- R²: die zuvor angegebene Bedeutung hat, und X für eine geeignete Abgangsgruppe, vorzugsweise für Halogen, insbesondere Chlor, Brom oder Iod, oder für Mesylat, Tosylat, Triflat oder 1-Imidazolyl, steht,
gegebenenfalls in Anwesenheit einer Base, umsetzt.

Das zuvor beschriebene Verfahren kann durch folgendes Formelschema beispielhaft erläutert werden:

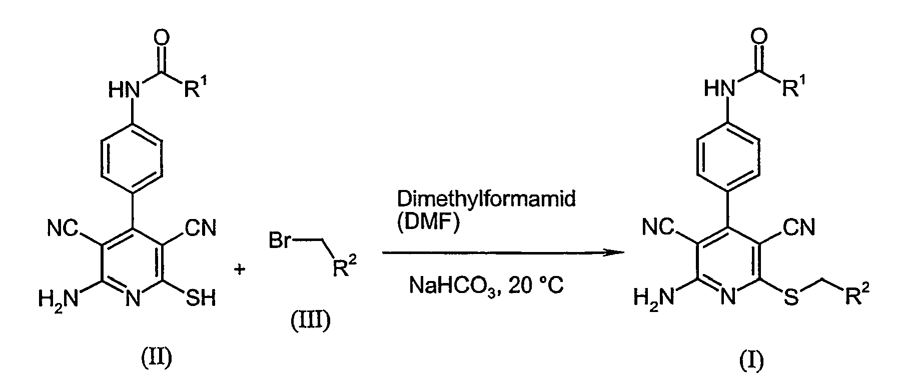

Als Lösemittel für das erfindungsgemäße Verfahren eignen sich alle organischen Lösemittel, die unter den Reaktionsbedingungen inert sind. Hierzu gehören Alkohole wie Methanol, Ethanol und Isopropanol, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Wasser ist als Lösemittel ebenso geeignet. Bevorzugt ist Dimethylformamid. Ebenso ist es möglich, Gemische der zuvor genannten Lösemittel einzusetzen.

Als Basen eignen sich die üblichen anorganischen oder organischen Basen. Hierzu gehören bevorzugt Alkalihydroxide wie beispielsweise Natrium- oder Kaliumhydroxid oder Alkalicarbonate wie Natrium- oder Kaliumcarbonat oder Alkalihydrogencarbonate wie Natrium- oder Kaliumhydrogencarbonat oder Alkalialkoholate wie Natrium- oder Kaliummethanolat, Natrium- oder Kaliumethanolat oder Kalium-tert.-butylat oder Amide wie Natriumamid, Lithium-bis-(trimethylsilyl)amid oder Lithiumdiisopropylamid oder metallorganische Verbindungen wie Butyllithium oder Phenyllithium oder aber Amine wie Triethylamin und Pyridin. Bevorzugt sind die Alkalicarbonate und -hydrogencarbonate.

Die Base kann hierbei in einer Menge von 1 bis 10 Mol, bevorzugt von 1 bis 5 Mol, insbesondere 1 bis 4 Mol, bezogen auf 1 Mol der Verbindungen der Formel (II) eingesetzt werden.

Die Reaktion erfolgt im allgemeinen in einem Temperaturbereich von -78°C bis zur +140°C, bevorzugt im Bereich von -78°C bis +40°C, insbesondere bei Raumtemperatur.

Die Umsetzung kann bei normalem, erhöhtem oder erniedrigtem Druck durchgeführt werden (z.B. im Bereich von 0,5 bis 5 bar). Im allgemeinen arbeitet man bei Normaldruck.

Die Verbindungen der Formeln (II) sind dem Fachmann an sich bekannt oder nach üblichen, literaturbekannten Methoden herstellbar.

Die Verbindungen der Formel (II) können auch aus Verbindungen der Formel (IV) durch Umsetzung mit einem Alkalisulfid hergestellt werden. Diese Herstellungsmethode kann durch folgendes Formelschema beispielhaft erläutert werden:

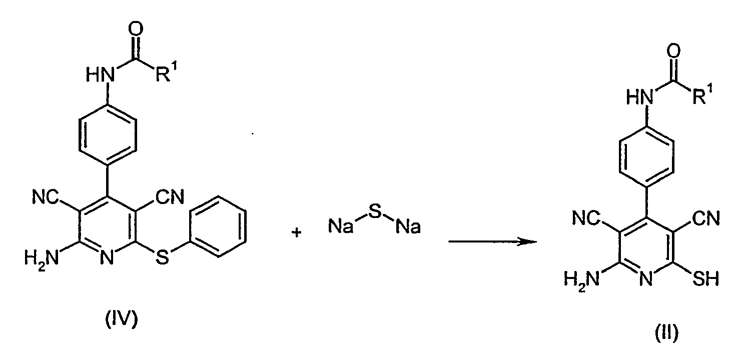

Als Alkalisulfid wird vorzugsweise Natriumsulfid in einer Menge von 1 bis 10 Mol, bevorzugt 1 bis 5 Mol, insbesondere 1 bis 4 Mol, bezogen auf 1 Mol der Verbindungen der Formel (IV) eingesetzt.

Als Lösungsmittel geeignet sind alle organischen Lösungsmittel, die unter den Reaktionsbedingungen inert sind. Hierzu gehören N,N-Dimethylformamid, N-Methylpyrrolidinon, Pyridin und Acetonitril. Besonders bevorzugt ist N,N-Dimethylformamid. Ebenso ist es möglich, Gemische der zuvor genannten Lösungsmittel einzusetzen.

Die Reaktion erfolgt im allgemeinen in einem Temperaturbereich von +20°C bis +140°C, bevorzugt im Bereich von +20°C bis +120°C, insbesondere bei +60°C bis +100°C.

Die Umsetzung kann bei normalem, erhöhtem oder erniedrigtem Druck durchgeführt werden (z.B. im Bereich von 0,5 bis 5 bar). Im allgemeinen arbeitet man bei Normaldruck.

Die Verbindungen der Formel (III) sind entweder kommerziell erhältlich, dem Fachmann bekannt oder nach üblichen Methoden herstellbar.

Die Verbindungen der Formel (IV) sind entweder kommerziell erhältlich, dem Fachmann bekannt oder nach üblichen Methoden herstellbar. Insbesondere kann auf die folgenden Druckschriften verwiesen werden, deren jeweiliger Inhalt durch Bezugnahme eingeschlossen wird:
- Kambe et al., Synthesis, 531-533 (1981);
- Elnagdi et al., Z. Naturforsch.47b, 572-578 (1991).

Überraschenderweise zeigen die Verbindungen der Formel (I) ein nicht vorhersehbares, wertvolles pharmakologisches Wirkspektrum und sind daher insbesondere zur Prophylaxe und/oder Behandlung von Erkrankungen geeignet.

Gegenüber dem Stand der Technik verfügen die erfindungsgemäßen Verbindungen der Formel (I) über verbesserte pharmakokinetische Eigenschaften, insbesondere über eine verbesserte Bioverfügbarkeit nach oraler Gabe.

Die Verbindungen der Formel (I) sind allein oder in Kombination mit einem oder mehreren anderen Wirkstoffen zur Prophylaxe und/oder Behandlung verschiedener Erkrankungen geeignet, so beispielsweise insbesondere von Erkrankungen des Herzkreislaufsystems (kardiovaskulären Erkrankungen). Geeignete Kombinationswirkstoffe sind insbesondere Wirkstoffe zur Behandlung von koronaren Herzkrankheiten wie beispielsweise insbesondere Nitrate, Betablocker, Calciumantagonisten oder Diuretika.

Im Sinne der vorliegenden Erfindung sind unter Erkrankungen des HerzkreislaufSystems bzw. kardiovaskulären Erkrankungen beispielsweise insbesondere die folgenden Erkrankungen zu verstehen: Koronare Herzkrankheit, Hypertonie (Bluthochdruck), Restenose wie z.B. Restenose nach Ballondilatation von peripheren Blutgefäßen, Arteriosklerose, Tachykardien, Arrhythmien, periphere und kardiale Gefäßerkrankungen, stabile und instabile Angina pectoris und Vorhofflimmern.

Weiterhin eignen sich die Verbindungen der Formel (I) beispielsweise insbesondere auch zur Reduktion des von einem Infarkt betroffenen Myokardbereichs.

Des weiteren eignen sich die Verbindungen der Formel (I) beispielsweise insbesondere zur Prophylaxe und/oder Behandlung von thromboembolischen Erkrankungen und Ischämien wie Myokardinfarkt, Hirnschlag und transitorischen ischämischen Attacken.

Weitere Indikationsgebiete, für das sich die Verbindungen der Formel (I) eignen, sind beispielsweise insbesondere die Prophylaxe und/oder Behandlung von Erkrankungen des Urogenitalbereiches, wie z.B. Reizblase, erektile Dysfunktion und weibliche sexuelle Dysfunktion, daneben aber auch die Prophylaxe und/oder Behandlung von inflammatorischen Erkrankungen, wie z.B. Asthma und entzündlichen Dermatosen, von neuroinflammatorischen Erkrankungen des Zentralnervensystems, wie beispielsweise Zustände nach Hirninfarkt, der Alzheimer-Erkrankung, weiterhin auch von neurodegenerative Erkrankungen, sowie von Schmerzzuständen und Krebs.

Ein weiteres Indikationsgebiet sind beispielsweise insbesondere die Prophylaxe und/oder Behandlung von Erkrankungen der Atemwege wie beispielsweise Asthma, chronische Bronchitis, Lungenemphysem, Bronchiektasien, zystische Fibrose (Mukoviszidose) und pulmonale Hypertonie.

Des weiteren kommen die Verbindungen der Formel (I) auch beispielsweise insbesondere für die Prophylaxe und/oder Behandlung von Leberfibrose und Leberzirrhose in Betracht.

Schließlich kommen die Verbindungen der Formel (I) beispielsweise insbesondere auch für die Prophylaxe und/oder Behandlung von Diabetes, insbesondere Diabetes mellitus, in Betracht.

Die vorliegende Erfindung betrifft auch die Verwendung der Verbindungen der Formel (I) zur Herstellung von Arzneimitteln zur Prophylaxe und/oder Behandlung der zuvor genannten Krankheitsbilder.

Die vorliegende Erfindung betrifft weiterhin ein Verfahren zur Prophylaxe und/oder Behandlung der zuvor genannten Krankheitsbilder mit den Verbindungen der Formel (I).

Die pharmazeutische Wirksamkeit der Verbindungen der Formel (I) lässt sich durch ihre Wirkung als Liganden an Adenosin-Al- und/oder Adenosin-A2b-Rezeptoren erklären.

Weiterer Gegenstand der vorliegenden Erfindung sind Arzneimittel, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Für die Applikation der Verbindungen der Formel (I) kommen alle üblichen Applikationsformen in Betracht, d.h. also oral, parenteral, inhalativ, nasal, sublingual, rektal, lokal wie beispielsweise bei Implantaten oder Stents, oder äußerlich wie beispielsweise transdermal. Bei der parenteralen Applikation sind insbesondere intravenöse, intramuskuläre oder subkutane Applikation beispielsweise als subkutanes Depot zu nennen. Bevorzugt ist die orale oder parenterale Applikation. Besonders bevorzugt ist die orale Applikation.

Hierbei können die Wirkstoffe allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen. Hierbei muss der Wirkstoff in einer solchen Menge vorliegen, dass eine therapeutische Wirkung erzielt wird. Im allgemeinen kann der Wirkstoff in einer Konzentration von 0,1 bis 100 Gew.-%, insbesondere 0,5 bis 90 Gew.-%, vorzugsweise 5 bis 80 Gew.-%, vorliegen. Insbesondere sollte die Konzentration des Wirkstoffs 0,5 - 90 Gew.-% betragen, d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin), Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten selbstverständlich auch Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserem oder Farbstoffen versetzt werden.

Im allgemeinen hat es sich als vorteilhaft erwiesen, bei parenteraler Applikation Mengen von etwa 0,1 bis etwa 10.000 µg/kg, vorzugsweise etwa 1 bis etwa 1.000 µg/kg, insbesondere etwa 1 µg/kg bis etwa 100 µg/kg Körpergewicht, zur Erzielung wirksamer Ergebnisse zu verabreichen. Bei oraler Applikation beträgt die Menge etwa 0,01 bis etwa 10 mg/kg, vorzugsweise etwa 0,05 bis etwa 5 mg/kg, insbesondere etwa 0,1 bis etwa 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die vorliegende Erfindung wird an den folgenden, nicht einschränkenden bevorzugten Beispielen veranschaulicht, die die Erfindung jedoch keinesfalls beschränken.

Die Prozentangaben der nachfolgenden Beispiele beziehen sich, sofern nicht anders angegeben, jeweils auf das Gewicht; Teile sind Gewichtsteile.

### A. Bewertung der physiologischen Wirksamkeit

### I. Nachweis der kardiovaskulären Wirkung

Narkotisierten Ratten wird nach Eröffnung des Brustkorbes das Herz schnell entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird. Die Frequenz des isoliert schlagenden Herzens wird rechnerisch aus der Anzahl der Kontraktionen pro Zeiteinheit ermittelt.

### II. Bestimmung des Adenosin-A1-, A2a-, A2b- und A3-Agonismus

### a) Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a, A2b transfiziert. Die Adenosin A1-Rezeptoren sind über Gi-Proteine und die Adenosin A2a- und A2b-Rezeptoren über Gs-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luciferase-Test wird mit dem Ziel hoher Sensitivität und Reproduzierbarkeit, geringer Varianz und guter Eignung für die Durchführung auf einem Robotersystem optimiert, indem mehrere Testparameter, wie z.B. Zelldichte, Dauer der Anzuchtphase und der Testinkubation, Forskolin-Konzentration, Medium-Zusammensetzung variiert werden. Zur pharmakologischen Charakterisierung der Zellen und zum roboter-gestützten Substanztest-Screening wird das folgende Testprotokoll verwendet:

Die Stammkulturen werden in DMEM/F12 Medium mit 10 % FCS (fötales Kälberserum) bei 37°C unter 5 % CO₂ gezüchtet und jeweils nach 2-3 Tagen 1:10 gesplittet. Testkulturen werden von 1000 bis 3000 Zellen pro Napf in 384-well Platten ausgesät und ca. 48 Stunden bei 37 °C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid 6 H₂O, 5 mM NaHCO₃, pH 7,4) ersetzt. Die in DMSO gelösten Substanzen werden dreimal 1:10 mit dieser physiologischen Kochsalzlösung verdünnt und zu den Testkulturen pipettiert (maximale DMSO-Endkonzentration im Testansatz: 0,5 %) So erhält man Substanzendkonzentrationen von beispielsweise 5 µM bis 5 nM. 10 Minuten später wird Forskolin zu den A1 Zellen zugegeben und anschließend werden alle Kulturen für vier Stunden bei 37 °C inkubiert. Danach wird zu den Testkulturen 35 µl Lösung, bestehend zu 50 % aus Lysereagenz (30 mM di-Natriumhydrogenphosphat, 10 % Glycerin, 3 % TritonX100, 25 mM TrisHCl, 2 mM Dithiotreitol (DTT), pH 7,8) und zu 50 % aus Luciferase Substrat Lösung (2,5 mM ATP, 0,5 mM Luciferin, 0,1 mM Coenzym A, 10 mM Tricin, 1,35 mM Magnesiumsulfat, 15 mM DTT, pH 7,8) zugegeben, ca. 1 Minute geschüttelt und die Luciferase-Aktivität mit einem Kamerasystem gemessen. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt (Klotz, K.N., Hessling, J., Hegler, J., Owman, C., Kull, B., Fredholm, B.B., Lohse, M.J., Comparative pharmacology of human adenosine receptor subtypes - characterization of stably transfected receptors in CHO cells, Naunyn Schmiedebergs Arch Pharmacol, 357 (1998), 1-9).

In der folgendenden Tabelle 1 sind Werte für die Rezeptorstimulation der Verbindung aus Beispiel 1 bei verschiedenen Konzentrationen an verschiedenen Adenosin-Rezeptor Subtypen angegeben.

**Tabelle 1: Adenosin-Rezeptorstimulation der Verbindung aus Beispiel 1 bei verschiedenen Konzentrationen**

| **Rezeptorsubtyp** | **Konzentration der Verbindung aus Beispiel 1** | | |
|---|---|---|---|
| | **10 nmol** | **1 nmol** | **0,3 nmol** |
| **A1** | **5** | **9** | **44** |
| **A2a** | **57** | **24** | **1** |
| **A2b** | **88** | **64** | **29** |

Angegeben sind die %-Werte des entsprechenden Referenzstimulus. Die Messwerte für den A2a- und den A2b-Rezeptor sind Angaben in Prozent der maximalen Stimulation durch NECA; die Messwerte für den A1-Rezeptor sind Angaben in Prozent nach direkter Vorstimulation der Adenylatcyclase durch 1 µmolar Forskolin (entspricht 100 %-Wert). A1-Agonisten zeigen entsprechend eine Abnahme der Luciferase-Aktivität (Messwert kleiner 100 %).

### b) Direkte Bestimmung des Adenosin-Agonismus über cAMP-Nachweis

Zellen der permanenten Linie CHO (Chinese Hamster Ovary) werden stabil mit der cDNA für die Adenosin-Rezeptor-Subtypen A1, A2a, A2b und A3 transfiziert. Die Bindung der Substanzen an die A2a- oder A2b-Rezeptorsubtypen wird bestimmt durch Messung des intrazellulären cAMP-Gehaltes in diesen Zellen mit einem konventionellen radioimmunologischen Assay (cAMP-RIA, IBL GmbH, Hamburg, Deutschland).

Im Falle der Wirkung der Substanzen als Agonisten kommt es als Ausdruck der Bindung der Substanzen zu einem Anstieg des intrazellulären cAMP-Gehaltes. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt (Klotz, K.N., Hessling, J., Hegler, J., Owman, C., Kull, B., Fredholm, B.B., Lohse, M.J., Comparative pharmacology of human adenosine receptor subtypes - characterization of stably transfected receptors in CHO cells, Naunyn Schmiedebergs Arch Phannacol, 357 (1998), 1-9).

Die Adenosin-Rezeptoren A1 und A3 sind an ein Gi-Protein gekoppelt, d.h. eine Stimulation dieser Rezeptoren führt zu einer Inhibition der Adenylatcyclase und somit zu einer Senkung des intrazellulären cAMP-Spiegels. Zur Identifizierung von A1/A3-Rezeptor-Agonisten wird die Adenylatcyclase mit Forskolin stimuliert. Eine zusätzliche Stimulation der A1/A3-Rezeptoren hemmt jedoch die Adenylatcyclase, so dass A1/A3-Rezeptor-Agonisten über einen vergleichsweise geringen Gehalt der Zelle an cAMP detektiert werden können.

Für den Nachweis einer antagonistischen Wirkung an Adenosin-Rezeptoren werden die mit dem entsprechenden Rezeptor transfizierten, rekombinanten Zellen mit NECA vorstimuliert und die Wirkung der Substanzen auf eine Reduktion des intrazellulären cAMP-Gehalts durch diese Vorstimulation untersucht. Als Referenzverbindung dient in diesen Experimenten XAC (xanthine amine congener), das mit hoher Affinität an alle Adenosinrezeptor-Subtypen bindet und eine antagonistische Wirkung besitzt (Müller, C.E., Stein, B., Adenosine receptor antagonists: structures and potential therapeutic applications, Current Pharmaceutical Design, 2 (1996) 501-530).

### III. Pharmakokinetische Untersuchungzen

Pharmakokinetische Daten wurden nach i.v.- sowie nach p.o.-Gabe verschiedener Substanzen als Lösung an Mäusen, Ratten und Hunden ermittelt. Hierzu wurden bis 24 Stunden nach Applikation Blutproben gesammelt. In den daraus gewonnenen Plasmaproben wurden die Konzentrationen der unveränderten Substanz mittels bioanalytischer Methoden (HPLC oder HPLC-MS) bestimmt. Anschließend wurden aus den so erhaltenen Plasmakonzentrations-Zeitverläufen pharmakokinetische Parameter ermittelt. In der folgenden Tabelle 2 sind die Bioverfügbarkeiten bei verschiedenen Species angegeben.

**Tabelle 2: Bioverfügbarkeiten nach oraler Gabe**

| | **Maus** | **Ratte** | **Hund** |
|---|---|---|---|
| Verbindung aus Beispiel 22 aus WO 00/125210 | nicht bestimmbar* | nicht bestimmbar* | 1,47 % |
| | (bei 3 mg/kg p.o) | (bei 10 mg/kg p.o) | (bei 1 mg/kg p.o) |
| Verbindung aus Beispiel 1 | 22,1 % | 4,6 % | 48,2% |
| | (bei 1 mg/kg p.o) | (bei 1 mg/kg p.o) | (bei 1 mg/kg p.o) |

| | | | |
|---|---|---|---|
| * Plasmaspiegel zu allen Messzeitpunkten unterhalb der Bestimmungsgrenze (<1 µg/l) | | | |

### B. Ausführungsbeispiele

### Beispiel 1

### N-(4-{2-amino-3,5-dicyano-6-[(2-pyridinylmethyl)sulfanyl]-4-pyridinyl}phenyl)acetamid

### 1. Stufe:

### N-[4-(2,2-Dicyanovinyl)phenyl]acetamid

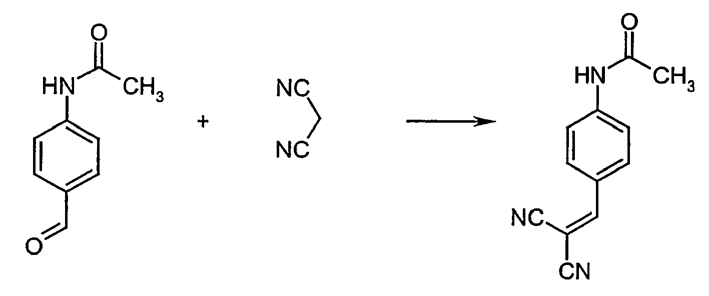

311.4 g (1.9 Mol) 4-Acetaminobenzaldehyd und 131 g (1.99 Mol) Malononitril werden in 1330 ml Ethanol vorgelegt und mit 6 ml Piperidin versetzt. Es wird 30 Minuten unter Rückfluss gerührt. Nach Abkühlen auf Raumtemperatur werden die Kristalle abgesaugt und getrocknet.
Ausbeute: 318 g (79 % d.Th.)
Massenspektrum: gesuchte Molmasse: 211; gefunden [M+H]⁺= 212

### 2. Stufe:

### N-{4-[2-amino-3,5-dicyano-6-(phenylsulfanyl)-4-pyridinyl]phenyl}acetamid

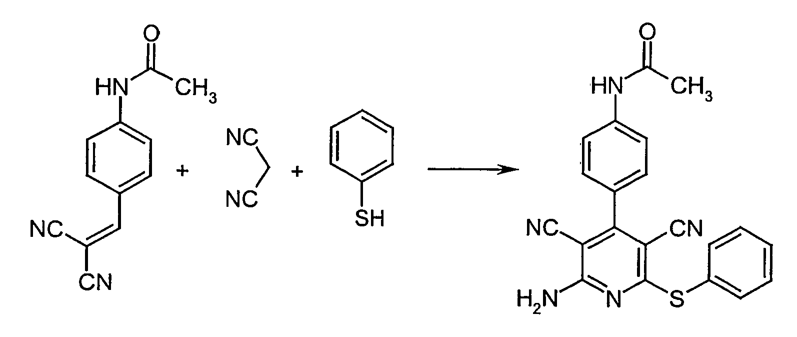

318 g (1.5 Mol) N-[4-(2,2-dicyanovinyl)phenyl]acetamid, 99 g (1.5 Mol) Malononitril und 166 g (1.5 Mol) Thiophenol werden in 2000 ml Ethanol vorgelegt und mit 6.7 ml Triethylamin versetzt. Es wird 2 Stunden unter Rückfluss gerührt, dabei findet Kristallisation statt. Nach dem Abkühlen auf Raumtemperatur wird das Produkt abgesaugt und i.V. getrocknet.
Ausbeute: 170.3 g (29 % d.Th.)
Massenspektrum: gesuchte Molmasse: 385; gefunden [M+H]⁺= 386

### 3. Stufe:

### N-[4-(2-amino-3,5-dicyano-6-sulfanyl-4-pyridinyl)phenyl]acetamid

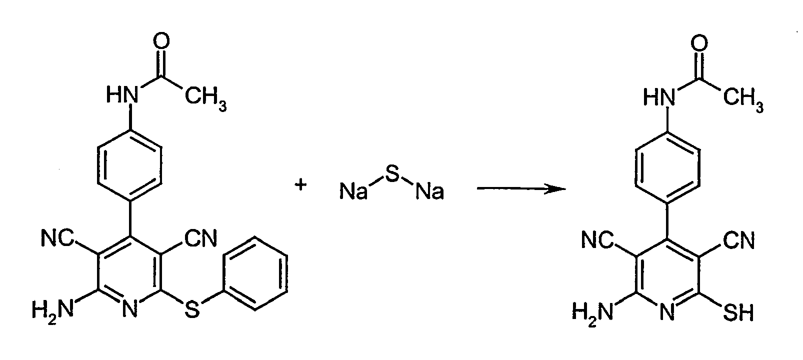

30.83 g (80 mmol) N-{4-[2-amino-3,5-dicyano-6-(phenylsulfanyl)-4-pyridinyl]-phenyl}-acetamid werden in 120 ml DMF unter Argon gelöst, 9.36 g (120 mmol) Natriumsulfid werden zugegeben und 2 Stunden bei 80°C gerührt. Dann wird eine Lösung von 20 ml 1N wässriger HCl in 44 ml Wasser bei 40 bis 65°C zugetropft, die dabei entstandenen Kristalle werden abgesaugt und mit Wasser gewaschen. Der Niederschlag wird in 200 ml Methanol suspendiert und 5 Minuten unter Rückfluss gerührt. Nach Abkühlen auf Raumtemperatur wird abgesaugt, mit Methanol und Diethylether gewaschen und i.V. getrocknet.
Ausbeute: 24.5 g (88 % d.Th.)
Massenspektrum: gesuchte Molmasse: 309; gefunden [M+H]⁺ = 310.1

### 4. Stufe:

### N-(4-{2-amino-3,5-dicyano-6-[(2-pyridinylmethyl)sulfanyl]-4-pyridinyl}phenyl)acetamid

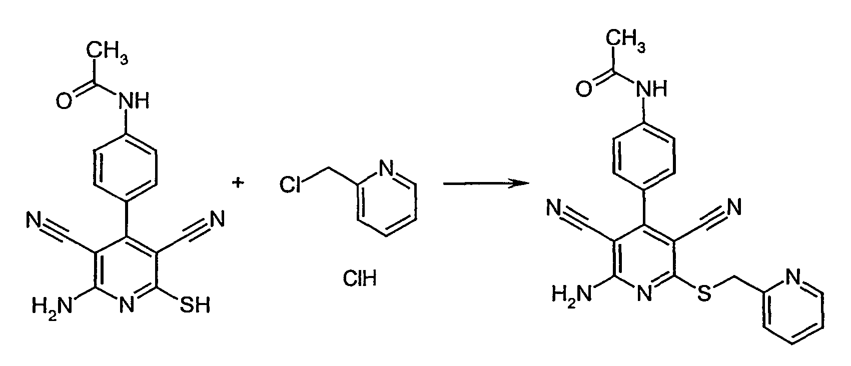

9.28 g (30 mmol) N-[4-(2-amino-3,5-dicyano-6-sulfanyl-4-pyridinyl)phenyl]-acetamid, 7.38 g (45 mmol) 2-Picolylchlorid Hydrochlorid und 10.08 g (120 mmol) Natriumhydrogencarbonat werden in 100 ml DMF bei Raumtemperatur gerührt. Nach 2 Stunden werden 100 ml Wasser bei 40 bis 50°C zugetropft. Nach Abkühlen auf Raumtemperatur werden die gelborangen Kristalle abgesaugt und i.V. getrocknet. Ausbeute: 10.42 g (86 % d.Th.)
Massenspektrum: gesuchte Molmasse: 400; gefunden [M+H]⁺= 401
¹H-NMR (300 MHz, DMSO-d₆): δ = 2.1 (s, 3H), 4.6 s (2H), 7.4 (dd, 1H), 7.45 (d, 1H), 7.65 (d, 2H), 7.75 (m, 3H), 8.1 (s breit, 2H), 8.5 (d, 1H), 10.25 (s, 1H).

### Beispiel 2 (Außerhalb der Erfindung)

### Methyl 4-(2-amino-3,5-dicyano-6-{[(2-methyl-1,3-thiazol-4-yl)methyl]sulfanyl}-4-pyridinyl)phenylcarbamat

### 1. Stufe:

### Methyl 4-(2-amino-3,5-dicyano-6-sulfanyl-4-pyridinyl)phenylcarbamat

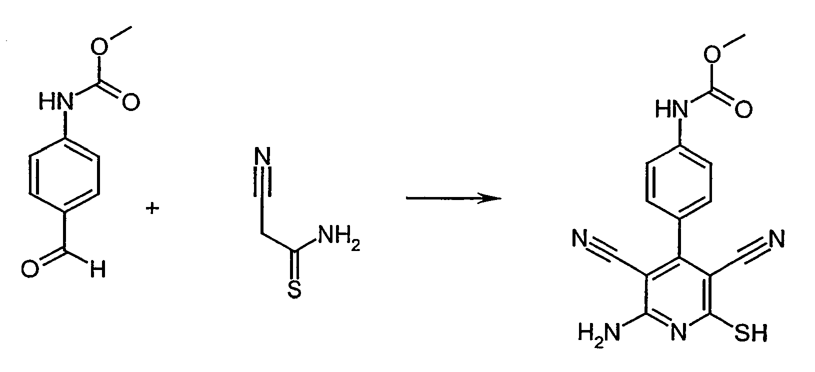

8.5 g (47.4 mmol) Methyl 4-formylphenylcarbamat (Witek et al, Journal f. Prakt. Chemie 321, 804-812 (1979)), 9.5 g (94.9 mmol) Cyanthioacetamid und 9.6 g (94.88 mmol) N-Methylmorpholin werden 3 Stunden in Ethanol unter Rückfluss erhitzt. Nach Eindampfen wird der Rückstand mit Dichlormethan/Methanol versetzt und filtriert. Das Filtrat wird nach Aufziehen auf Kieselgur durch Chromatographie an Kieselgel (Elutionsmittel: Diclormethan/Methanol 100:2 bis 100:6) gereinigt. Die Produktfraktionen werden vereinigt und eingedampft. Der Eindampfrückstand wird in 200 ml 1 N wässriger Natronlauge gelöst und filtriert. Das Filtrat wird mit 300 ml 1 N wässriger Salzsäure versetzt, der entstandene Niederschlag wird abgesaugt und i.V. getrocknet.
Ausbeute: 2.7 g (17 % d.Th.)
Massenspektrum: gesuchte Molmasse: 325; gefunden [M+H]⁺= 326
¹H-NMR (200 MHz, DMSO-d₆): δ = 3.7 s (3H), 7.4 (d, 2H), 7.6 (d, 2H), 8.1 (s breit, 2H), 10.0 (s, 1H)

### 2. Stufe:

### Methyl 4-(2-amino-3,5-dicyano-6-{[(2-methyl-1,3-thiazol-4-yl)methyl]sulfanyl}-4-pyridinyl)phenylcarbamat

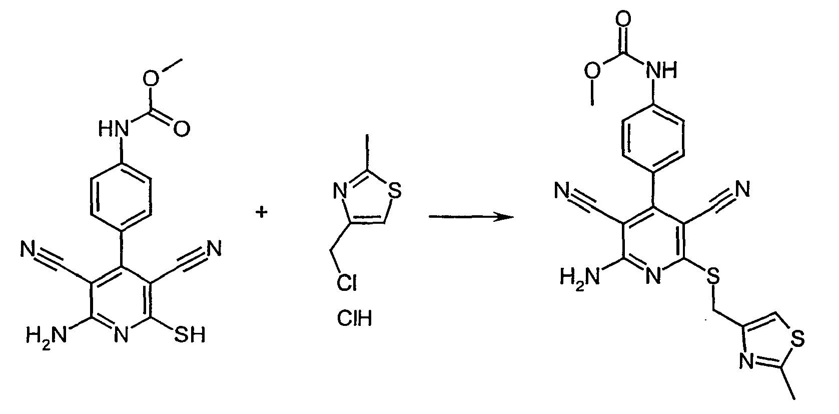

32.5 mg (0.1 mmol) Methyl 4-(2-amino-3,5-dicyano-6-sulfanyl-4-pyridinyl)phenylcarbamat und 27.6 mg (0.15 mmol) 4-(Chloromethyl)-2-methyl-1,3-thiazol Hydrochlorid werden zusammen mit 33.6 (0.4 mmol) Natriumhydrogencarbonat in 0.4 ml DMF über Nacht geschüttelt. Die Reaktionsmischung wird filtriert und durch präparative HPLC gereinigt.
Säule: Nucleosil 5C18 Nautilus, 5µm, 20X50 mm,
Vorsäule: Gromsil ODS 4 HE 15µm 10x20 mm.
Flussrate: 25 ml/min.
Gradient (A = Acetonitril, B=Wasser + 0.3% Trifluoressigsäure):

| | |
|---|---|
| 0 min | 10% A; |
| 2 min | 10% A; |
| 6 min | 90% A; |
| 7.00 min | 90% A; |
| 7.10 min | 10% A; |
| 8 min | 10% A. |

Detektion: 220 nm. Injektionsvolumen: 600 µl

Die Produktfraktion wird im Vakuum eingedampft.
Ausbeute: 15.8 mg (36 % d.Th.)
Massenspektrum: gesuchte Molmasse: 436; gefunden [M+H]⁺= 437

### Verwendete Abkürzungen:

- DMF: Dimethylformamid
- DMSO: Dimethylsulfoxid
- HEPES: 2-[4-(2-Hydroxyethyl)piperazino]ethansulfonsäure
- d. Th.: der Theorie
- HPLC: Hochdruck-, Hochleistungsflüssigchromatographie
- NMR: Kernresonanzspektroskopie
- RT: Raumtemperatur
- Tris: 2-Amino-2-(hydroxymethyl)-1,3-propandiol
- i. V.: im Vakuum

## Patentansprüche

1. Verbindungen der Formel (I) 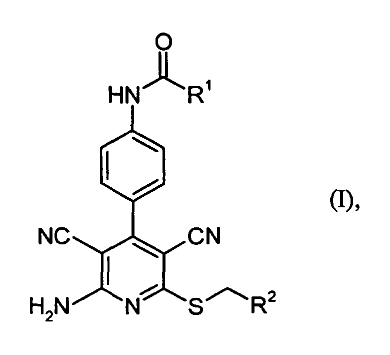 worin
R¹ (C₁-C₄)-Alkyl, (C₁-C₄)-Alkoxy, Mono- oder Di-(C₁-C₄)-alkylamino bedeutet
und
R² Pyridyl der durch Halogen, Amino oder (C₁-C₄)-Alkyl substituiert sein kann, bedeutet
und ihre Salze, Hydrate, Hydrate der Salze und Solvate.

2. Verbindungen der Formel (I) nach Anspruch 1,
worin
R¹ Methyl, Ethyl, n-Propyl, Isopropyl, n-Butyl, sec-Butyl, Isobutyl oder tert.-Butyl bedeutet
und
R² 2-Pyridyl, der durch Chlor, Amino oder Methyl substituiert sein kann, bedeutet
und ihre Salze, Hydrate, Hydrate der Salze und Solvate.

3. Verbindung nach Anspruch 1 oder 2 mit der folgenden Struktur 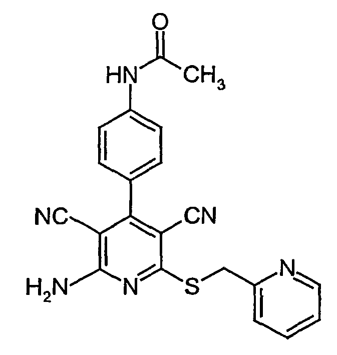 und ihre Salze, Hydrate, Hydrate der Salze und Solvate.

4. Verfahren zur Herstellung der Verbindungen der Formel (I), wie in Anspruch 1 definiert, **dadurch gekennzeichnet, dass** man
Verbindungen der Formel (II) 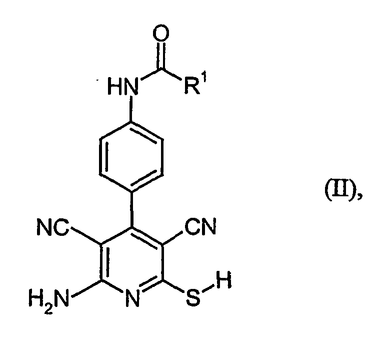 worin
R¹ die in Anspruch 1 angegebene Bedeutung hat,
mit Verbindungen der Formel (III)
R²-CH₂-X (III),
worin
R² die in Anspruch 1 angegebene Bedeutung hat, und X für eine geeignete Abgangsgruppe steht,
umsetzt.

5. Verbindungen der Formel (I), wie in Anspruch 1 definiert, zur Prophylaxe und/oder Behandlung von Erkrankungen.

6. Arzneimittel, enthaltend mindestens eine Verbindung der Formel (I), wie in Anspruch 1 definiert, und mindestens einen weiteren Hilfsstoff.

7. Arzneimittel, enthaltend mindestens eine Verbindung der Formel (I), wie in Anspruch 1 definiert, und mindestens einen weiteren Wirkstoff.

8. Verwendung von Verbindungen der Formel (I), wie in Anspruch 1 definiert, zur Herstellung von Arzneimitteln zur Prophylaxe und/oder Behandlung von Erkrankungen des Herzkreislaufsystems (kardiovaskulären Erkrankungen).

9. Verwendung von Verbindungen der Formel (I), wie in Anspruch 1 definiert, zur Herstellung von Arzneimitteln zur Prophylaxe und/oder Behandlung von Erkrankungen des Urogenitalbereichs und Krebs.

10. Verwendung von Verbindungen der Formel (I), wie in Anspruch 1 definiert, zur Herstellung von Arzneimitteln zur Prophylaxe und/oder Behandlung von inflammatorischen und neuroinflammatorischen Erkrankungen, neurodegenerativen Erkrankungen und Schmerzzuständen.

11. Verwendung von Verbindungen der Formel (I), wie in Anspruch 1 definiert, zur Herstellung von Arzneimitteln zur Prophylaxe und/oder Behandlung von Erkrankungen der Atemwege, von Leberfibrose und Leberzirrhose und Diabetes.

## Claims

1. Compounds of the formula (I) 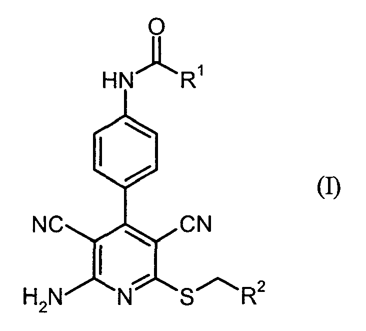 in which
R¹ denotes (C₁-C₄)-alkyl, (C₁-C₄)-alkoxy, mono- or di-(C₁-C₄)-alkylamino,
and
R² denotes pyridyl, which may be substituted by halogen, amino or (C₁-C₄)-alkyl,
and their salts, hydrates, hydrates of the salts and solvates.

2. Compounds of the formula (I) according to Claim 1,
in which
R¹ denotes methyl, ethyl, n-propyl, isopropyl, n-butyl, sec-butyl, isobutyl or tert-butyl,
and
R² denotes 2-pyridyl, which may be substituted by chlorine, amino or methyl,
and their salts, hydrates, hydrates of the salts and solvates.

3. Compound according to Claim 1 or 2 having the following structure 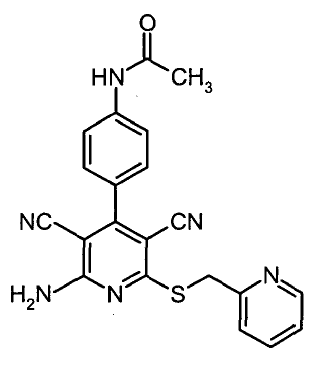 and its salts, hydrates, hydrates of the salts and solvates.

4. Process for preparing the compounds of the formula (I) as defined in Claim 1, **characterized in that**
compounds of the formula (II) 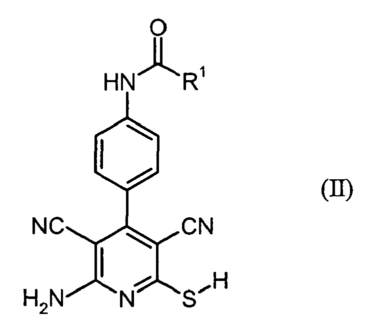 in which
R¹ has the meaning given in Claim 1,
are reacted with compounds of the formula (III)
R²-CH₂-X (III)
in which
R² has the meaning given in Claim 1 and X represents a suitable leaving group.

5. Compounds of the formula (I), as defined in Claim 1, for the prophylaxis and/or treatment of diseases.

6. Medicament comprising at least one compound of the formula (I), as defined in Claim 1, and at least one additional auxiliary substance.

7. Medicament comprising at least one compound of the formula (I), as defined in Claim 1, and at least one additional active compound.

8. Use of compounds of the formula (I), as defined in Claim 1, for producing medicaments for the prophylaxis and/or treatment of cardiovascular diseases.

9. Use of compounds of the formula (I), as defined in Claim 1, for producing medicaments for the prophylaxis and/or treatment of diseases of the urogenital region and cancer.

10. Use of compounds of the formula (I), as defined in Claim 1, for producing medicaments for the prophylaxis and/or treatment of inflammatory and neuroinflammatory diseases, neurodegenerative diseases and pain.

11. Use of compounds of the formula (I), as defined in Claim 1, for producing medicaments for the prophylaxis and/or treatment of diseases of the airways, of liver fibrosis and liver cirrhosis and diabetes.

## Revendications

1. Composés de formule (I) : 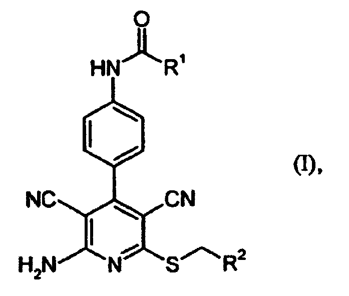 dans laquelle
R¹ représente un reste alkyle en C₁ à C₄, alkoxy en C₁ à C₄, mono- ou di(alkyle en C₁ à C₄)amino,
et
R² représente un reste pyridyle qui peut être substitué par un radical halogéno, amino ou alkyle en C₁ à C₄,
et leurs sels, leurs hydrates, les hydrates des sels et leurs produits de solvatation.

2. Composés de formule (I) suivant la revendication 1, où
R¹ désigne un reste méthyle, éthyle, n-propyle, isopropyle, n-butyle, sec.-butyle, isobutyle ou tertiobutyle,
et
R² désigne un reste 2-pyridyle qui peut être substitué par un radical chloro, amino ou méthyle,
et leurs sels, leurs hydrates, les hydrates des sels et leurs produits de solvatation.

3. Composé suivant la revendication 1 ou 2, de formule suivante: 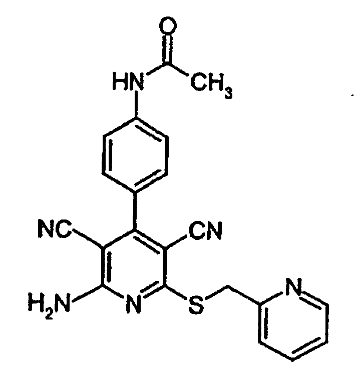 et ses sels, ses hydrates, les hydrates des sels et ses produits de solvatation.

4. Procédé de production des composés de formule (I), tels que définis dans la revendication 1, **caractérisé en ce qu'**on fait réagir des composés de formule (II) : 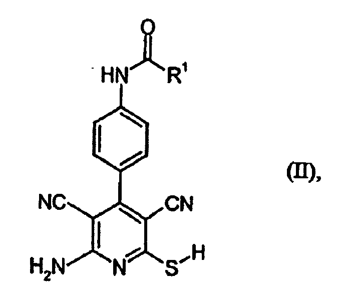 dans laquelle :
R¹ a la définition indiquée dans la revendication 1,
avec des composés de formule (III) :
**R²-CH₂-X** **(III),**
dans laquelle
R² a la définition indiquée dans la revendication 1 et X représente un groupe partant convenable.

5. Composés de formule (I), tels que définis dans la revendication 1, destinés à la prophylaxie et/ou au traitement de maladies.

6. Médicament, contenant au moins un composé de formule (I), tel que défini dans la revendication 1, et au moins une autre substance auxiliaire.

7. Médicament, contenant au moins un composé de formule (I), tel que défini dans la revendication 1, et au moins une autre substance active.

8. Utilisation de composés de formule (I), tels que définis dans la revendication 1, pour la préparation de médicaments destinés à la prophylaxie et/ou au traitement de maladies du système cardio-vasculaire (maladies cardio-vasculaires).

9. Utilisation de composés de formule (I), tels que définis dans la revendication 1, pour la préparation de médicaments destinés à la prophylaxie et/ou au traitement de maladies de l'appareil génito-urinaire et du cancer.

10. Utilisation de composés de formule (I), tels que définis dans la revendication 1, pour la préparation de médicaments destinés à la prophylaxie et/ou au traitement de maladies inflammatoires et neuro-inflammatoires, de maladies neurodégénératives et d'états douloureux.

11. Utilisation de composés de formule (I), tels que définis dans la revendication 1, pour la préparation de médicaments destinés à la prophylaxie et/ou au traitement de maladies des voies respiratoires, de la fibrose hépatique et de la cirrhose du foie, et du diabète.
